(19) Europäisches Patentamt

European Patent Office

Office européen des brevets

(11) **EP 3 441 077 A1**

(12) **EUROPEAN PATENT APPLICATION**
published in accordance with Art. 153(4) EPC

(43) Date of publication:
**13.02.2019 Bulletin 2019/07**

(21) Application number: **16897545.6**

(22) Date of filing: **07.04.2016**

(51) Int Cl.:
*A61K 36/738* (2006.01)      *A61K 36/66* (2006.01)
*A61K 36/282* (2006.01)      *A61K 35/60* (2006.01)
*A61K 9/48* (2006.01)          *A61P 19/08* (2006.01)
*A61P 37/04* (2006.01)

(86) International application number:
**PCT/CN2016/078650**

(87) International publication number:
**WO 2017/173610 (12.10.2017 Gazette 2017/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicants:
• **Beijing Lu De Kai Qi Co., Ltd.
Beijing 100000 (CN)**

• **Lu, Wei
Beijing 100000 (CN)**

(72) Inventor: **LU, Wei
Beijing 100000 (CN)**

(74) Representative: **Dr. Gassner & Partner mbB
Wetterkreuz 3
91058 Erlangen (DE)**

(54) **PHARMACEUTICAL PRODUCT FOR TREATING ANKYLOSING SPONDYLITIS AND COMBINED IMMUNE DEFECT, AND PREPARATION AND APPLICATION THEREOF**

(57)  A pharmaceutical product for treating ankylosing spondylitis for combined immunodeficiency which is prepared from a *Rosa roxbunghii* extract, an *Artemisia argyi* extract, a *hippocampus* extract, and a *Corydalis Rhizoma* extract in a weight ratio of (1-10):(0.5-2):1:(1-10) by supercritical fluid extraction. The pharmaceutical product facilitates in vivo dissolution and human body absorption.

**Description**

**Technical Field**

The present invention relates to the field of medicine, and more particularly, to a pharmaceutical product for anti-ankylosing spondylitis for combined immunodeficiency, preparation methods and use thereof.

**Background Art**

Ankylosing spondylitis (AS for short) is a disease with an inflammation of the sacroiliac joint and spinal joint being as the main symptom, and is strongly associated with HLA-B27. Certain microorganisms, such as Klebsiella, have a common antigen with the susceptible's own tissue and can trigger an abnormal immune response. Ankylosing spondylitis is a chronic inflammatory disease characterized by fibrosis and ossification of the large joints of the extremities, annulus fibrosus of intervertebral disc and the adjacent connective tissue thereof. Ankylosing spondylitis belongs to the category of rheumatism and is a kind of seronegative spondyloarthropathy. The etiology of this disease is still unclear. It is a chronic disease with the spine being as the main lesion, involving the sacroiliac joint, causing spinal stiffness and fibrosis, resulting in different degrees of lesions of eye, lung, muscle and bone, and is an autoimmune disease. Modern medical treatment is mainly based on pharmaceutical product therapy such as a non-steroid antiinflammatory pharmaceutical product, etc., aiming at relieving symptoms, controlling the condition and preventing joint deformities. However, there are certain adverse effects in long-term use, especially severe symptoms of gastrointestinal discomfort, severely, resulting in ulcer bleeding, perforation, myeloid suppression, abnormal liver function, etc.

Chinese Patent with Publication No. CN102091137B discloses a Chinese medicine preparation for treating ankylosing spondylitis, which improves microcirculation by improving immunity so as to relieve symptoms and delay the progression of the disease. Although this method can improve the patient's immunity to a certain extent, it cannot repair the immune system that was destroyed before treatment, and cannot guarantee the immune balance in the internal environment, which leads to the recurrence of the disease and is unfavorable to the patient's health.

How to effectively and safely treat ankylosing spondylitis and prevent the recurrence of the disease has become one of the key directions in the art.

**Summary of the Invention**

In view of the deficiencies of the prior art, the object of the present invention is to provide a pharmaceutical product for anti-ankylosing spondylitis for combined immunodeficiency which is beneficial to improve the clinical symptom of ankylosing spondylitis, can stimulate cellular immune regulatory factors, enhance cellular immune activity, repair the damaged immunity due to the prior treatment and the like, improve immune hypofunction or inhibit immune hyperfunction, regulate the internal environment, maintain the immune function balance, and prevent the recurrence of ankylosing spondylitis.

In order to achieve the above object, the present invention provides a pharmaceutical product for treating ankylosing spondylitis and combined immunodeficiency, which is prepared from a *Rosa roxbunghii* extract, an *Artemisia argyi* extract, a *hippocampus* extract, and a *Corydalis Rhizoma* extract in a weight ratio of (1-10):(0.5-2):1:(1-10).

Preferably, the *Artemisia argyi* is *Artemisia absinthium,* and the *Rosa roxbunghii, Artemisia argyi, hippocampus,* and *Corydalis Rhizoma* are all wild.

*Rosa roxbunghii,* also known as siberian rose or roxburgh rose, is the fruit of Rosa roxburghii, which is a perennial deciduous shrub belonging to Rosaceae. It has cool nature , and is sweet, sour, and slightly astringent, and it can clear away heat and promote fluid production, invigorate the stomach and promote digestion, and relieve summer-heat. The main components of *Rosa roxbunghii* extract according to the present invention comprises vitamin C, vitamin P, vitamin B, vitamin E, folic acid, carotene, superoxide dismutase, peroxidase, polyphenol, polyphenols, malic acid, citric acid, flavonoid, polysaccharide, tannic acid, various amino acids and various trace elements. It has been found that the active ingredients in different ratios can achieve the object of the present invention, and the *Rosa roxbunghii* extract prepared by the preparation method according to the present invention is most effective.

*Artemisia argyi,* also known as folium artemisiae argyi, felon herb, Chinese mugwort or Jia Ai, is a dry leave of *Artemisia argyi Levi.et Vant.* belonging to *Compositae.* It is pungent and bitter, has warm nature, has slight toxicity and can eliminate cold to stop pain and warming meridian to stop bleeding. The *Artemisia argyi* extract according to the present invention is preferably prepared by extraction of the whole grass of woormwood (or *Artemisia absinthium*). The main component of this extract is volatile oil, and the oil contains terpinenol-4, β-caryophyllene, artemisia alcohol, linalool, camphorae, borneol, cineol (eucalyptol), trans-traved carveol, α-terpineol, etc., and polysaccharides, tannic acid, thujone, phellandrene, β- sitosterol, 5,7-dihydroxy-6,3',4'-trimethyl flavonoid. It has been found that the active ingredients in different ratios can achieve the object of the present invention, and the *Artemisia argyi* extract prepared by the preparation method of the present invention is most effective.

*Hippocampus,* also known as pair sea horse, *Hkuda* or water horse, is the the whole after removing the internal organs of *Hippocampus kelloggi, Hippocampus histrix, Hkuda, Hippocampus trimaculatus* or *Hippocampus zosterae* belonging to *Syngnathidae.* It is sweet and has warm naturer, and can warm kidney and tonify yang, dissipate binds and disperse swelling. The *hippocampus* extract according to the present invention is prepared by extracting the hippocampus in which the viscera and gray-black outer membrane has been removed. The main components of this extract are amino acid, protein, fatty acid, steride and trace element, as well as stearic acid, cholesterol, cholesterdiol, acetylcholinesterase, cholinesterase and protease. It has been found that the active ingredients in different ratios can achieve the object of the present invention, and the *hippocampus* extract prepared by the preparation method of the present invention is most effective.

*Corydalis Rhizoma,* also known as yanhu, xuanhusuo, and *rhizoma corydalis,* is a tuber of *Corydalis yanhusuo W.T. Wang* belonging to *Papaveraceae.* It is pungent and bitter and has warm nature, and is non-toxic, and can activate blood, reinforce qi, and relieve pain. The *Corydalis Rhizoma* extract according to the present invention is prepared by extracting the tuber of *Corydalis Rhizoma,* and its main components of this extract are alkaloid, mucilage, resin, volatile oil, polysaccharide, hydroxystreptomycin, stigmasterol, oleic acid, linoleic acid, linolenic acid, fumaric acid, 10-nonacosanol. The alkaloid mainly includes corydalis A (d-corydaline, fumarine), corydalis B (dl-tetrahydropalmatine), corydalis C (protopine), corydalis D (1-tetrahydrocoptisine), corydalis E (dl-tetrahydrocoptisine), corydalis F (1-tetrahydrocolumbamine), corydalis G (corybulbine), corydalis H, corydalis I, corydalis J, corydalis K, corydalis L, corydalis M ($\alpha$-Alloc-ryptopine, $\beta$-Homochelidonine), coptisine, dehydrocorydalis A (dehydrofumarine, dehydrocorydaline), corydalmine, dehydrocorydalmine, columbamine, leonticine, dihydrosanguinarine, and dehydronanteine. It has been found that the active ingredients in different ratios can achieve the object of the present invention, and the *Corydalis Rhizoma* extract prepared by the preparation method of the present invention is most effective.

It has been found that the pharmaceutical product for anti-ankylosing spondylitis for combined immunodeficiency according to the present invention which is prepared from a *Rosa roxbunghii* extract, an *Artemisia argyi* extract, a *hippocampus* extract, and a *Corydalis Rhizoma* extract in a weight ratio of (1-10):(0.5-2):1:(1-10) has the following synergistic effects:

(a) supplementing a variety of nutrients, anti-inflammation, analgesia, relieving and improving symptoms in patient with ankylosing spondylitis, and increasing said patient's quality of life, such as relieving pain, increasing appetite, analgesia and replenishing blood;
(b) having the function of bacteriostasis, sterilization, resisting virus, having strong anti-oxidation and hydroxyl radical scavenging ability, and enhancing the ability of patients with ankylosing spondylitis to fight disease;
(c) replenishing liver and kidney, joining sinew and bone, regulating the blood vessels, relaxing tendons and dredging collaterals, lubricating joint, dispelling wind-cold, and accelerating the discharge of toxins in the body, which is beneficial to improve the clinical symptoms of ankylosing spondylitis;
(d) stimulating cellular immune regulatory factor, enhancing cellular immune activity, also regulating internal environment and restoring normal physiological balance to ensure that the treatment of ankylosing spondylitis can be carried out normally;
(e) repairing the damaged immunity due to the prior treatment and the like, improving immune hypofunction or inhibiting immune hyperfunction, regulating the internal environment, maintaining the immune function balance, and preventing the recurrence of ankylosing spondylitis.

It has been found that the weight ratio of *Rosa roxbunghii* extract, *Artemisia argyi* extract, *hippocampus* extract, and *Corydalis Rhizoma* extract is preferably 5:2:1:5.

Preferably, the preparation of the pharmaceutical product is a capsule.

Administration in the form of capsule is advantageous for ensuring that the pharmaceutical product for treating ankylosing spondylitis for combined immunodeficiency of the present invention can be administered accurately and conveniently, which is advantageous for control.

Another object of the present invention is to provide a preparation method of the pharmaceutical product for treating ankylosing spondylitis for combined immunodeficiency, characterized in that the method comprises the preparation *of Rosa roxbunghii* extract capsule, the preparation of *Artemisia argyi* extract capsule, the preparation of *hippocampus* extract capsule, and the preparation of *Corydalis Rhizoma* extract capsule; the preparation *of Rosa roxbunghii* extract capsule, the preparation of *Artemisia argyi* extract capsule, the preparation of *hippocampus* extract capsule, and the preparation of *Corydalis Rhizoma* extract capsule all include:

S1, screening and washing raw materials, and softening the clean raw materials by quenching under vacuum;
S2, subjecting the raw material softened in step S1 to supercritical fluid extraction, and separating to obtain an extract liquor and a residue;

S3, pulverizing and grinding the residue obtained in step S2;

S4, spray drying the extract liquor obtained in step S2 and adding the pulverized and ground residue obtained in step S3 during the spray drying to obtain a dried raw material extract; and S5, filling a formulated amount of the raw material extract obtained in step S4 into a capsule shell to obtain a capsule.

The present invention employs the softening method by the method of quenching under vacuum, which rapidly inhales the water by the action of negative pressure so as to accelerate the softening of the medicine, ensures softening of the medicine with water at normal temperature, shortens the infiltration time, reduces the loss and damage of active ingredients and decreases the mildew of medicine.

The present invention employs supercritical fluid extraction technology for extracting medicinal plants, wherein the fluid is carbon dioxide and the extraction temperature is 30-40°C,which can avoid the volatilization of components of volatile oil ,etc. during the extraction process, and can maximally maintain the effective bioactive components of medicinal plants. At the same time, there is no solvent residue since the organic solvent is not applicable in the process, which ensures the natural biological activity of the raw materials, environmental protection, safety and no side effects.

The present invention employs spray drying for quickly drying the raw material extract liquor of the invention, and the obtained product has fine and uniform particle size, good fluidity, increased specific surface area, which is beneficial to its dissolution in the body and the absorption by the human body. The raw material extract liquor is spray-dried to obtain fine particle. Since the particle contains a viscous substance such as sugar, protein or starch, when the residue of the raw material is added during spray drying of the raw material extract, the residue of the raw material can adsorb the spray-dried raw material extract liquor, and the spray-dried raw material extract liquor has strong binding force with the residue of the raw material, which can prevent the occurrence of sticking. At the same time, the raw material medicine is fully utilized, and no other external auxiliary materials are added, which are conducive to maintain its natural activity.

The capsule prepared by the above preparation is safety and has no side effects. The extraction process does not destroy the biological active component of the original plant, and the biological active component can be fully made into an extract having a large specific surface area, which is beneficial to its dissolution in the body and the absorption by the human body. At the same time, using this method can avoid the sticking phenomenon or the like which occurs during the production process.

Preferably, it has been found that in step S1, the method of quenching under vacuum is performed at the temperature of 10-40 °C and the pressure of -0.1 to -0.09 MPa; in step S2, the fluid for supercritical fluid extraction is carbon dioxide, and the extraction temperature is 30-40 °C;

in step S4, the spray drying temperature is 40-80 °C, the drying time is 3-60 s, and the raw material extract has a particle size of 10-60 μm; and

in step S5, the capsule shell is a natural plant capsule shell.

Another object of the present invention is also to provide a preparation method of the pharmaceutical product for treating ankylosing spondylitis for combined immunodeficiency, and the method comprises the preparation *of Rosa roxbunghii* extract capsule, the preparation of *Artemisia argyi* extract capsule, the preparation of *hippocampus* extract capsule, and the preparation of *Corydalis Rhizoma* extract capsule; the preparation *of Rosa roxbunghii* extract capsule comprises:

S1, screening and washing *Rosa roxbunghii,* and softening the clean *Rosa roxbunghii* by quenching under vacuum; wherein the quenching under vacuum is performed at the temperature of 10-40 °C and the pressure of -0.1 to -0.09 MPa;

S2, subjecting the *Rosa roxbunghii* softened in step S1 to supercritical fluid extraction, and separating to obtain a *Rosa roxbunghii* extract liquor and a *Rosa roxbunghii* residue; wherein the fluid for supercritical fluid extraction is carbon dioxide, and the extraction temperature is 30-40 °C;

S3, pulverizing and grinding the *Rosa roxbunghii* residue obtained in step S2;

S4, spray drying the *Rosa roxbunghii* extract liquor obtained in step S2 and adding the pulverized and ground *Rosa roxbunghii* residue obtained in step S3 during the spray drying to obtain a dried *Rosa roxbunghii* extract; wherein the spray drying temperature is 40-80 °C, the drying time is 3-60 s, and the *Rosa roxbunghii* extract has a particle size of 10-60 μm; and

S5, filling 1-10 parts *of Rosa roxbunghii* extract obtained in step S4 into a natural plant capsule shell to obtain a *Rosa roxbunghii* extract capsule;

the preparation of *Artemisia argyi* extract capsule comprises:

S1, screening and washing *Artemisia argyi,* and softening the clean whole grass of *Artemisia argyi* by quenching under vacuum; wherein the quenching under vacuum is performed at the temperature of 10-40 °C and the pressure of -0.1 to -0.09 MPa;

S2, subjecting the *Artemisia argyi* softened in step S 1 to supercritical fluid extraction, and separating to obtain an *Artemisia argyi* extract liquor and an *Artemisia argyi* residue; wherein the fluid for supercritical fluid extraction is carbon dioxide, and the extraction temperature is 30-40 °C;

S3, pulverizing and grinding the *Artemisia argyi* residue obtained in step S2;

S4, spray drying the *Artemisia argyi* extract liquor obtained in step S2 and adding the pulverized and ground *Artemisia argyi* residue obtained in step S3 during the spray drying to obtain a dried *Artemisia argyi* extract; wherein the spray drying temperature is 40-80 °C, the drying time is 3-60 s, and the *Artemisia argyi* extract has a particle size of 10-60 μm; and

S5, filling 0.5-2 parts of *Artemisia argyi* extract obtained in step S4 into a natural plant capsule shell to obtain an *Artemisia argyi* extract capsule;

the preparation of *Hippocampus* extract capsule comprises:

S1, screening and washing *Hippocampus,* and softening the clean *Hippocampus* with viscera and gray-black outer membrane being removed by quenching under vacuum; wherein the quenching under vacuum is performed at the temperature of 10-40 °C and the pressure of -0.1 to -0.09 MPa;

S2, subjecting the *Hippocampus* softened in step S1 to supercritical fluid extraction, and separating to obtain a *Hippocampus* extract liquor and a *Hippocampus* residue; wherein the fluid for supercritical fluid extraction is carbon dioxide, and the extraction temperature is 30-40 °C;

S3, pulverizing and grinding the *Hippocampus* residue obtained in step S2;

S4, spray drying the *Hippocampus* extract liquor obtained in step S2 and adding the pulverized and ground *Hippocampus* residue obtained in step S3 during the spray drying to obtain a dried *Hippocampus* extract; wherein the spray drying temperature is 40-80 °C, the drying time is 3-60 s, and the *Hippocampus* extract has a particle size of 10-60 μm; and

S5, filling 1 part of *Hippocampus* extract obtained in step S4 into a natural plant capsule shell to obtain a *Hippocampus* extract capsule;

the preparation of *Corydalis Rhizoma* extract capsule comprises:

S1, screening and washing *Corydalis Rhizoma,* and softening the clean tuber of *Corydalis Rhizoma* by quenching under vacuum; wherein the quenching under vacuum is performed at the temperature of 10-40 °C and the pressure of -0.1 to -0.09 MPa;

S2, subjecting the *Corydalis Rhizoma* softened in step S1 to supercritical fluid extraction and separating to obtain a *Corydalis Rhizoma* extract liquor and a *Corydalis Rhizoma* residue; wherein the fluid for supercritical fluid extraction is carbon dioxide, and the extraction temperature is 30-40 °C;

S3, pulverizing and grinding the *Corydalis Rhizoma* residue obtained in step S2;

S4, spray drying the *Corydalis Rhizoma* extract liquor obtained in step S2 and adding the pulverized and ground *Corydalis Rhizoma* residue obtained in step S3 during the spray drying to obtain a dried *Corydalis Rhizoma* extract; wherein the spray drying temperature is 40-80 °C, the drying time is 3-60 s, and the *Corydalis Rhizoma* extract has a particle size of 10-60 μm; and

S5, filling 1-10 parts of *Corydalis Rhizoma* extract obtained in step S4 into a natural plant capsule shell to obtain a *Corydalis Rhizoma* extract capsule; and

the *Artemisia argyi* is *Artemisia absinthium,* and the *Rosa roxbunghii, Artemisia argyi, hippocampus,* and *Corydalis Rhizoma* are all wild.

In the pharmaceutical products for treating ankylosing spondylitis for combined immunodeficiency according to the present invention, the *Rosa roxbunghii* extract capsule, the *Artemisia argyi* extract capsule, the *hippocampus* extract capsule, and the *Corydalis Rhizoma* extract capsule are separately prepared. On the one hand, the mutual interference of the four plants can be avoided during the treatment, which is beneficial to ensure the quality of the product. On the other hand, the administration can be varied according to the needs during the treatment so as to apply to different types of patients.

Another object of the present invention is also to provide a use of a pharmaceutical product for treating ankylosing spondylitis for combined immunodeficiency in the manufacture of a pharmaceutical product for preventing or treating ankylosing spondylitis.

Further object of the present invention is to provide a use of a pharmaceutical product for treating ankylosing spondylitis for combined immunodeficiency prepared by the preparation method in the manufacture of a pharmaceutical product for preventing or treating ankylosing spondylitis.

The following beneficial effects are obtained by using the above technical solutions:

1. The pharmaceutical product for treating ankylosing spondylitis for combined immunodeficiency according to the present invention has the following synergistic effect: bacteriostasis, sterilization, having strong anti-oxidation and hydroxyl radical scavenging ability, replenishing liver and kidney, joining sinew and bone, regulating the blood vessels, relaxing tendons and dredging collaterals, lubricating joint, dispeling wind-cold, accelerating the discharge of toxins in the body, which is beneficial to improve the clinical symptoms of ankylosing spondylitis; stimulating cellular immune regulatory factor, enhancing cellular immune activity; repairing the damaged immunity due to the prior treatment and the like, improving immune hypofunction or inhibiting immune hyperfunction, regulating the internal environment, maintaining the immune function balance, and preventing the recurrence of ankylosing spondylitis.

2. In the preparation process of the pharmaceutical product for treating ankylosing spondylitis for combined immunodeficiency according to the present invention, the effective bioactive component of the medicinal plant can be extracted to the greatest extent without using organic solvents and destructive conditions, and the composition is safe and has no side effects. Meanwhile, the effective bioactive component can also be made into the particle with a large specific surface area, which is beneficial to the absorption in human body.

3. In the preparation process of the pharmaceutical product for treating ankylosing spondylitis for combined immunodeficiency according to the present invention, the residue of the raw material is added during the spray drying of the raw material extract liquor, which is beneficial to solve the sticking phenomenon of the raw material extract during the production process together with the conventional anti-sticking operation, and to utilize the raw material medicine fully to maintain its natural activity.

4. The pharmaceutical product for treating ankylosing spondylitis for combined immunodeficiency according to the present invention is in the form of a capsule, and thus the administration is precise and convenient, which is advantageous for preservation and stability.

**Examples**

In the examples, the *Rosa roxbunghii* is selected from the wild *Rosa roxbunghii*. The *Artemisia argyi* is selected from the wild *Artemisia absinthium*. The *hippocampus* is selected from the wild *Hippocampus histrix*. The *Corydalis Rhizoma* is selected from the wild *Corydalis Rhizoma.*

The *Rosa roxbunghii* is screened and washed, and the clean *Rosa roxbunghii* is softened by quenching under vacuum at the temperature of 10-40 °C and the pressure of -0.1 to -0.09 MPa. The softened *Rosa roxbunghii* is subject to the supercritical fluid extraction carried out at 30-40 °C using a carbon dioxide fluid, and a *Rosa roxbunghii* extract liquor and a *Rosa roxbunghii* residue is obtained by separating. The *Rosa roxbunghii* residue is pulverized, ground and passed through a 200-300 mesh sieve. The *Rosa roxbunghii* extract liquor is sprayed onto the sieved *Rosa roxbunghii* residue by spray drying at a temperature of 40 to 80 °C for a drying time of 3 to 60 s to obtain a *Rosa roxbunghii* extract having a particle size of 10-60 μm. The *Rosa roxbunghii* extract is filled into a natural plant capsule shell by a conventional capsule filling method to obtain a *Rosa roxbunghii* extract capsule.

The whole grass of *Artemisia argyi* is screened and washed, and the clean whole grass of *Artemisia argyi* is softened by quenching under vacuum at the temperature of 10-40 °C and the pressure of -0.1 to -0.09 MPa. The softened *Artemisia argyi* is subject to the supercritical fluid extraction carried out at 30-40 °C using a carbon dioxide fluid, and an *Artemisia argyi* extract liquor and an *Artemisia argyi* residue was obtained by separating. The *Artemisia argyi* residue was pulverized, ground and passed through a 200-300 mesh sieve. The *Artemisia argyi* extract liquor was sprayed onto the sieved *Artemisia argyi* residue by spray drying at a temperature of 40 to 80 °C for a drying time of 3 to 60 s to obtain an *Artemisia argyi* extract having a particle size of 10-60 μm. The *Artemisia argyi* extract was filled into a natural plant capsule shell by a conventional capsule filling method to obtain an *Artemisia argyi* extract capsule.

The *Hippocampus* with viscera and gray-black outer membrane being removed is screened and washed, and the clean *Hippocampus* was softened by quenching under vacuum at the temperature of 10-40 °C and the pressure of -0.1 to -0.09 MPa. The softened *Hippocampus* is subject to the supercritical fluid extraction carried out at 30-40 °C using a carbon dioxide fluid, and a *Hippocampus* extract liquor and a *Hippocampus* residue was obtained by separating. The *Hippocampus* residue was pulverized, ground and passed through a 200-300 mesh sieve. The *Hippocampus* extract liquor was sprayed onto the sieved *Hippocampus* residue by spray drying at a temperature of 40 to 80 °C for a drying time of 3 to 60 s to obtain a *Hippocampus* extract having a particle size of 10-60 μm. The *Hippocampus* extract was filled into a natural plant capsule shell by a conventional capsule filling method to obtain a *Hippocampus* extract capsule.

The tuber of *Corydalis Rhizoma* was screened and washed, and the clean *Corydalis Rhizoma* is softened by quenching under vacuum at the temperature of 10-40 °C and the pressure of -0.1 to -0.09 MPa. The softened *Corydalis Rhizoma* is subject to the supercritical fluid extraction carried out at 30-40 °C using a carbon dioxide fluid, and a *Corydalis Rhizoma* extract liquor and a *Corydalis Rhizoma* residue was obtained by separating. The *Corydalis Rhizoma* residue was pulverized, ground and passed through a 200-300 mesh sieve. The *Corydalis Rhizoma* extract liquor was sprayed onto the sieved *Corydalis Rhizoma* residue by spray drying at a temperature of 40 to 80 °C for a drying time of 3 to 60 s to obtain a *Corydalis Rhizoma* extract having a particle size of 10-60 $\mu$m. The *Corydalis Rhizoma* extract was filled into a natural plant capsule shell by a conventional capsule filling method to obtain a *Corydalis Rhizoma* extract capsule.

The *Rosa roxbunghii* extract capsule, the *Artemisia argyi* extract capsule, the *hippocampus* extract capsule, and the *Corydalis Rhizoma* extract capsule are simultaneously administered in a certain ratio when using pharmaceutical product for treating ankylosing spondylitis for combined immunodeficiency according to the present invention.

Table 1 The pharmaceutical product for treating ankylosing spondylitis for combined immunodeficiency according to the present invention

| Extract capsule | Example | Example 1 | Example 2 | Example 3 | Example 4 | Example 5 |
|---|---|---|---|---|---|---|
| *Rosa roxbunghii* extract capsule | Mass of *Rosa roxbunghii* extract (mg) | 1000 | 300 | 1500 | 600 | 400 |
| | Type of natural plant capsule shell | 0# | 3# | 0# | 0# | 0# |
| *Artemisia argyi* extract capsule | Mass of *Artemisia argyi* extract (mg) | 400 | 150 | 150 | 75 | 200 |
| | Type of natural plant capsule shell | 0# | 3# | 3# | 3# | 3# |
| *hippocampus* extract capsule | Mass of *hippocampus* extract (mg) | 200 | 300 | 150 | 100 | 200 |
| | Type of natural plant capsule shell | 3# | 3# | 3# | 3# | 3# |
| *Corydalis Rhizoma* extract capsule | Mass of *Corydalis Rhizoma* extract (mg) | 1000 | 300 | 1200 | 1000 | 600 |
| | Type of natural plant capsule shell | 0# | 3# | 0# | 0# | 0# |

Comparative example 1. Toxicology assay

1. Test materials: The *Rosa roxbunghii* extract, the *Artemisia argyi* extract, the *hippocampus* extract, and the *Corydalis Rhizoma* extract are all prepared in the same manner as in Example 1. The *Rosa roxbunghii* extract, the *Artemisia argyi* extract, the *hippocampus* extract, and the *Corydalis Rhizoma* extract are respectively subjected to a water bath at 70 °C for 1 hour, soaked for 12 hours, and concentrated for testing.
2. Test animals:
The healthy Kunming mice provided by the Experimental Animal Center of the National Institute for the Control of Pharmaceutical and Biological Products are selected.
3. Mouse bone marrow polychromatic erythrocyte micronucleus assay

The assay was carried out via oral gavage and the administration was performed twice at intervals of 24 hr. 50 mice weighing 25-30 g are randomly divided into 5 groups according to body weight, 10 in each group, half male and half female. The cyclophosphamide (cp) at a dose of 50 mg/kg•bw was used as a positive control, and the distilled water was used as a negative control. The doses of the pharmaceutical product for treating ankylosing spondylitis for combined immunodeficiency according to the present invention are 2.50, 5.00, and 10.00 g/kg•bw, respectively, which were adjusted to the desired concentration with distilled water. The animals were sacrificed by cervical dislocation six hours after the last administration of the pharmaceutical product for treating ankylosing spondylitis for combined immunodeficiency according to the present invention. The bone marrow of sternum was diluted with calf serum, smeared, fixed in methanol, and stained with Giemsa. Under the light microscope, 1000 polychromatic erythrocytes (PRC) were measured per animal, and the incidence of micronucleus was determined by the permillage of micronucleus-containing PRC and statistical process is performed. The statistical results about the effects of the pharmaceutical product for treating ankylosing spondylitis for combined immunodeficiency according to the present invention on the incidence of bone marrow micronucleus in mice are shown in Table 2.

Table 2 Statistical results of mouse bone marrow polychromatic erythrocyte micronucleus assay

| Gender | Dose (g/kg•bw) | Number of animals | Number of cells examined | Number of micronucleus | Micronucleus rate (‰) |
|---|---|---|---|---|---|
| Male | 0.00 | 5 | 5000 | 9 | 1.8 |
| | 2.50 | 5 | 5000 | 7 | 1.4 |
| | 5.00 | 5 | 5000 | 8 | 1.6 |
| | 10.00 | 5 | 5000 | 10 | 2.0 |
| | 50 mg/kg•bw (cp) | 5 | 5000 | 167 | 33.4* |
| Female | 0.00 | 5 | 5000 | 8 | 1.6 |
| | 2.50 | 5 | 5000 | 8 | 1.6 |
| | 5.00 | 5 | 5000 | 8 | 1.6 |
| | 10.00 | 5 | 5000 | 10 | 2.0 |
| | 50 mg/kg•bw (cp) | 5 | 5000 | 150 | 30.0* |
| * Extremely significant difference from the negative control group (P<0.01). | | | | | |

It can be seen from table 2 that the micronucleus rate of each dose group of the pharmaceutical product for treating ankylosing spondylitis for combined immunodeficiency according to the present invention is small has no significantly difference compared with the negative control group, while the difference between the cyclophosphamide group and the negative control group is extremely significant (P<0.01). The pharmaceutical product for treating ankylosing spondylitis for combined immunodeficiency according to the present invention does not affect the micronucleus rate of mouse bone marrow polychromatic erythrocytes.

4. Sperm abnormality assay in mice
50 sexually mature male mice weighing 30-35 g is randomly divided into 5 groups. The cyclophosphamide at a dose of 40 mg/kg•bw was used as a positive control, and the distilled water was used as a negative control. The doses of the pharmaceutical product for treating ankylosing spondylitis for combined immunodeficiency according to the present invention is 1.25, 2.50, and 5.00 g/kg•bw, respectively, which were adjusted to the desired concentration with distilled water. The mice were administered via oral gavage once a day for 5 days, and the animals were sacrificed 30 days after the last gavage. The epididymis is sectioned and stained with eosin. Five animals were measured in each group and 1000 spermatozoa with intact structural were measured per animal, by which the incidence of sperm abnormality (in percentage) was calculated and statistical process is performed. The statistical results of the effects of the pharmaceutical product for treating ankylosing spondylitis for combined immunodeficiency according to the present invention on the incidence of sperm abnormality in mice are shown in Table 3.

Table 3 Statistical results of sperm abnormality assay in mice

| Dose (g/kg•bw) | Number of animals | Number of sperms examined | Number of abnormal sperms | Rate of sperm abnormality (%) |
|---|---|---|---|---|
| 0.00 | 5 | 5000 | 100 | 2.00 |
| 2.50 | 5 | 5000 | 85 | 1.88 |
| 5.00 | 5 | 5000 | 89 | 1.78 |
| 10.00 | 5 | 5000 | 100 | 2.00 |
| 40 mg/kg•bw (cp) | 5 | 5000 | 487 | 9.74* |
| * Extremely significant difference from the negative control group (P<0.01). | | | | |

It can be seen from table 3 that the pharmaceutical product for treating ankylosing spondylitis for combined immunodeficiency according to the present invention does not significantly affect the incidence of sperm abnormality in the mice, and there is no significant difference between the respective dose groups and the negative control group, while the difference between the cyclophosphamide group and the negative control group is extremely significant (P<0.01). Therefore, the pharmaceutical product for treating ankylosing spondylitis for combined immunodeficiency according to the present invention does not affect the sperm abnormality in the mice.

The pharmaceutical products for treating ankylosing spondylitis for combined immunodeficiency according to the present invention prepared in Examples 2-5 were also subjected to the same toxicological assay, and the results were consistent with that of the pharmaceutical product for treating ankylosing spondylitis for combined immunodeficiency according to the present invention prepared in Example 1.

Comparative example 2. Immunomodulatory assay

1. Test materials: The *Rosa roxbunghii* extract capsule, the *Artemisia argyi* extract capsule, the *hippocampus* extract capsule, and the *Corydalis Rhizoma* extract capsule are all prepared in the same manner as in Example 1. The recommended amount for oral administration in human is 2.6 g/day, which is equivalent to 0.043 g/kg•bw calculated at 60 kg body weight per person (one capsule for each variety). The equivalent dose in the mouse is equivalent to 10 times the recommended amount for the human body, that is, 0.43 g/kg•bw (medium dose) of product per day. One dose group was set for high and low doses, respectively: 1.30 g/kg•bw (high dose) of the stereotyped product and 0.043 g/kg•bw of the stereotyped product (low dose). The samples are formulated in sterilized water and the samples for control group are replaced with sterilized water. After continuous gavage for 30 days, various immune indicators were measured. Since the *Rosa roxbunghii* extract capsule, the *Artemisia argyi* extract capsule, and the *hippocampus* extract capsule are all insoluble in water, these capsules were soaked 3 times with 10 times the recommended amount of hot water (70-80 °C), 1 hour each time, and then are concentrated by evaporation on a rotary evaporator (50-60 °C) to a concentration of 30 times the recommended dose, and then diluted proportionally for gavage.

2. Test animals:

75 healthy male Kunming mice weighing 18-22 g provided by the Experimental Animal Center of the National Institute for the Control of Pharmaceutical and Biological Products are selected and divided into 4 groups with 15 per group for the dose groups and 30 for the control group to carry out the delayed type hypersensitivity and antibody forming cell assays. 75 healthy male Kunming mice weighing 18-22 g provided by the Experimental Animal Center of the National Institute for the Control of Pharmaceutical and Biological Products were selected and divided into 4 groups with 15 per group for the dose groups and 30 for the control group to carry out carbon clearance assay.

3. Delayed type hypersensitivity assay (DTH) (toe incrassation)

The mice were intraperitoneally injected with 0.2 mL of SRBC with 2% (v/v, prepared with physiological saline, 0.2 mL/mouse). Four days after sensitization, the thickness of the left and right footpads was measured, and the same position was measured three times and the results were averaged. Then, 20% (v/v, prepared with physiological saline) of SRBC (20 μL/mouse) was injected subcutaneously at the measurement site, and the thickness of the left and right footpads were measured 24 hours after the injection, and the same position was measured three times and the results were averaged. The degree of DTH was expressed by the difference in the thickness of the footpad before and after the injection (swelling degree of the footpad). The statistical results of the effects of the pharmaceutical product for treating ankylosing spondylitis for combined immunodeficiency according to the present invention on delayed type hypersensitivity (DTH) in mice are shown in Table 4.

Table 4. Statistical results of delayed type hypersensitivity assay (DTH)

| Group | Number of animals | Swelling degree of the footpad (mm) |
|---|---|---|
| Control | 30 | 0.423 ± 0.109 |
| Low dose | 15 | 0.663 ± 0.101 |
| Medium dose | 15 | 0.737 ± 0.112 |
| High dose | 15 | 0.723 ± 0.111 |

After the mice were orally administered with different doses of the pharmaceutical product for treating ankylosing spondylitis for combined immunodeficiency according to the present invention for 30 days, the results were statistically processed. As seen from Table 4, as for the swelling degree of the footpad, there was an extremely significant difference between the low, medium and high dose groups and the control group (P<0.01). In other words, the pharmaceutical product for treating ankylosing spondylitis for combined immunodeficiency according to the present invention can significantly enhance the delayed type hypersensitivity in mice.

4. Antibody forming cell assay (modified Jerne plaque assay)

Mice are immunized by intraperitoneal injection of 0.2 mL of SRBC with 2% (v/v, prepared with physiological saline, 0.2 mL per mouse). After 5 days, the mice were sacrificed, and the spleen is taken out and shredded and prepared into a cell suspension using Hank's solution, in which the cells were washed, centrifuged twice, and suspended in 5 mL of Hank's solution. After heating and dissolving the surface medium (agarose), it is mixed with an equal amount of double Hank's solution and dispensed into small tubes with 0.5 mL per tube. 50 μL SRBC of 10% (v/v, prepared with SA solution) and 20 μL spleen cell suspension are added to the tubes. After quickly mixing, the mixture is poured onto a slide coated with agarose. After the agar solidified, the slide was placed horizontally on the holder and incubated in a carbon dioxide incubator for 1.5 hr. The complement diluted with SA buffer (1:10) is added to the groove of the slide holder. After incubation for 1.5 hr, the number of hemolysis plaques is counted. The statistical results of the effects of the pharmaceutical product for treating ankylosing spondylitis for combined immunodeficiency according to the present invention on the number of antibody forming cells are shown in Table 5.

Table 5 Statistical results of antibody forming cell assay

| Group | Number of animals | Number of hemolysis plaques ($\times 10^3$/whole spleen) |
|---|---|---|
| Control | 22 | 111.2 ± 50.8 |
| Low dose | 11 | 111.2 ± 42.6 |
| Medium dose | 13 | 121.6 ± 52.3 |
| High dose | 12 | 119.5 ± 94.1 |

After the mice were orally administered with different doses of the pharmaceutical product for treating ankylosing spondylitis for combined immunodeficiency according to the present invention for 30 days, the results were statistically processed. As seen from Table 5, as for the swelling degree of the footpad, there was no extremely significant difference between the low, medium and high dose groups and the control group (P>0.05). In other words, the pharmaceutical product for treating ankylosing spondylitis for combined immunodeficiency according to the present invention does not affect the number of antibody forming cells in mice.

5. Carbon clearance assay in mice

The tail vein of the mice was injected with Indian ink which is 4 times diluted with physiological saline at a dose of 0.1 mL per 10 g body weight, and timing was started immediately after the injection. 20 μL of blood was taken from the angular vein 2 and 10 minutes after the injection respectively, added to 2 mL of 0.1% $Na_2CO_3$ solution, and shaken well. The 0.1% $Na_2CO_3$ solution was used as a blank control, and the optical density value (OD) was measured at a wavelength of 600 nm using a 721 spectrophotometer. The mice were sacrificed, the liver and spleen were taken out and weighed (body weight is expressed in m, liver weight is expressed in $m_1$, spleen weight is expressed in $m_2$), and the phagocytic index a was calculated as follows:

$$K = \frac{\lg OD_1 - \lg OD_2}{t_2 - t_1} \qquad (\text{Formula } 1-1)$$

$$a = \frac{m}{m_2 + m_1} \times \sqrt[3]{k} \qquad (\text{Formula } 1-2)$$

The statistical results of the effects of the pharmaceutical product for treating ankylosing spondylitis for combined immunodeficiency according to the present invention on the monocyte-macrophage phagocytosis in mice are shown in Table 6.

Table 6 Statistical results of carbon clearance assay in mice

| Group | Number of animals | Number of hemolysis plaques ($\times 10^3$/whole spleen) |
|---|---|---|
| Control | 26 | 6.01 $\pm$ 0.60 |
| Low dose | 15 | 6.13 $\pm$ 0.40 |
| Medium dose | 14 | 5.87 $\pm$ 0.56 |
| High dose | 14 | 5.91 $\pm$ 0.60 |

After the mice were orally administered with different doses of the pharmaceutical product for treating ankylosing spondylitis for combined immunodeficiency according to the present invention for 30 days, and the results were statistically processed. As seen from Table 6, as for the swelling degree of the footpad, there was no extremely significant difference between the low, medium and high dose groups and the control group (P>0.05). In other words, the pharmaceutical product for treating ankylosing spondylitis for combined immunodeficiency according to the present invention does not affect the monocyte-macrophage carbon clearance in mice.

As seen from Tables 4-6, after the mice were orally administered with different doses of the pharmaceutical product for treating ankylosing spondylitis for combined immunodeficiency according to the present invention for 30 days, the pharmaceutical product for treating ankylosing spondylitis for combined immunodeficiency according to the present invention can significantly enhance the delayed type hypersensitivity in mice, and does not affect the number of antibody forming cells and the monocyte-macrophage carbon clearance in mice. Therefore, the pharmaceutical product for treating ankylosing spondylitis for combined immunodeficiency according to the present invention has the effect of enhancing cellular immune function.

The pharmaceutical products for treating ankylosing spondylitis and combined immunodeficiency according to the present invention prepared in Examples 2-5 are also subjected to the same immunomodulatory assay, and the results are consistent with that of the pharmaceutical product for treating ankylosing spondylitis for combined immunodeficiency according to the present invention prepared in Example 1.

Comparative example 3. Hygienic inspection of preparations

With reference to GB4789-94 and GB4789.15-94, the pharmaceutical product for treating ankylosing spondylitis for combined immunodeficiency according to the present invention prepared in Example 1 was tested for coliform bacteria, total number of colonies, pathogenic bacteria, mold, and yeast count. A set of samples newly produced and a set of samples placed at 25 °C/60% RH for 3 years were tested separately, and both sets of samples were samples for the same production batch. Ten samples were taken in parallel for each set of samples. The hygienic inspection results are shown in Table 7.

Table 7 Statistical results of hygienic inspection of preparations

| Capsule / Test items | Rosa roxbunghii extract capsule | | Artemisia argyi extract capsule | | hippocampus extract capsule | | Corydalis Rhizoma extract capsule | |
|---|---|---|---|---|---|---|---|---|
| | 0 day | 3 years | 0 day | 3 years | 0 day | 3 years | 0 day | 3 years |
| Coliform bacteria (MPN/100 g) | <30 | <30 | <30 | <30 | <30 | <30 | <30 | <30 |

| | | | | | | | | |
|---|---|---|---|---|---|---|---|---|
| Total number of colonies (CFU/g) | <10 | <10 | <10 | <10 | <10 | <10 | <10 | <10 |
| Staphylococcus aureus | Not detected | Not detected | Not detected | Not detected | Not detected | Not detected | Not detected | Not detected |
| Streptococcus hemolyticus | Not detected | Not detected | Not detected | Not detected | Not detected | Not detected | Not detected | Not detected |
| Shigella | Not detected | Not detected | Not detected | Not detected | Not detected | Not detected | Not detected | Not detected |
| salmonella | Not detected | Not detected | Not detected | Not detected | Not detected | Not detected | Not detected | Not detected |
| Mold (CFU/g) | Not detected | Not detected | Not detected | Not detected | Not detected | Not detected | Not detected | Not detected |
| Yeast (CFU/g) | Not detected | Not detected | Not detected | Not detected | Not detected | Not detected | Not detected | Not detected |

As can be seen from Table 7, the pharmaceutical products for treating ankylosing spondylitis for combined immunodeficiency newly produced and placed at 25 °C/60% RH for 3 years passed hygienic inspection. The pharmaceutical products for treating ankylosing spondylitis for combined immunodeficiency according to the present invention prepared in Examples 2-3 were also subjected to the same hygienic inspection, and the results are consistent with that of the pharmaceutical product for treating ankylosing spondylitis and combined immunodeficiency according to the present invention prepared in Example 1.

Comparative example 4. Clinical trial

Clinical trials were performed in patients with ankylosing spondylitis. The criteria for inclusion are as follows:

(1) The New York Standard for Ankylosing Spondylitis revised in 1984 is met.
(2) The diagnostic criteria for the "renal deficiency and cold syndrome" of Ankylosing Spondylitis in Traditional Chinese Medicine is met. It is obtained by reference to the "Guidelines for Clinical Research of New Pharmaceutical products for Treating Arthralgia" and combined with the clinical experience of Professor Jiao Shude and Professor Yan Xiaoping. The syndrome of renal deficiency and cold syndrome is: pain and stiffness of waist, buttocks, and crotch, and the resulting pain of knee, leg, shoulder, elbow and other joints, and fear of cold and be fond of warm, cool limbs, being desirous for getting heat, limited pitch, poor activity, even stiffness or deformation of lumbar spine, being incapable of walking, sitting and lying, lassitude, asthenia, sweating, loose stools, clear urine in large amounts, or at the same time being cold of scrotum in male, being cold and leucorrhea in female; thin white or thick white coating on the tongue, deep wiry pulse or small wiry pulse, and weak cubit pulse; meeting the inflammatory back pain (IBP) criteria and the new axial SpA criteria published by International Spondylarthropathy (SpA) Assessment (ASAS) Working Group in 2009 , that is, if the patient whose low back pain lasts at least 3 months and age of onset being less than 45 years old meeting any of the following criteria, axial SpA (prototype thereof is AS) can be confirmed: where the imaging results are present, the imaging suggests sacroiliitis and the patient has more than one clinical features of SpA; where the imaging results are absent, the patient is HLA-B27 positive and has two other clinical features of SpA. The five criteria for IBP are: age of onset <40 years old; insidious onset; improvement after exercise; no improvement after rest; and nocturnal pain (improvement after getting up). If the patient meets 4 of them, he or she can be confirmed to have IBP (sensitivity 77.0%, specificity 91.7%). Imaging suggests sacroiliitis means that: MRI of sacroiliac joint suggests active (acute) inflammation (clear bone marrow edema or osteitis), highly suggests SpA-related sacroiliitis; or X-ray suggests sacroiliitis (same as the New York standard revised in 1984). SpA clinical features: IBP; arthritis; tendonitis (heel); uveitis; finger (toe) inflammation; psoriasis; Crohn's disease/ulcerative colitis; good response to NSAIDs treatment (completely disappeared or significantly improved pain 24-48 hours after administration); a family history of SpA (referring to one or two generations of relatives with any of AS, psoriasis, acute uveitis, reactive arthritis, inflammatory bowel disease); HLA-B27 positive; and elevated CRP.

(3) Age is between 18 and 45 years old.

(4) Excluded people: pregnant or lactating female patients, or patients who cannot tolerate this pharmaceutical product, or patients with severe primary diseases concerning heart, cerebrovascular, liver, kidney and hematopoietic system diseases, and mental illness; patients who have advanced severe joint deformity, disability, or lose their labor ability; patients with other seronegative spondyloarthropathies or other rheumatisms at the same time; patients who have recently used hormonal pharmaceutical products; patients who have peptic ulcers within 1 month before this study; and patients who are currently participating or participated other clinical observations for treating ankylosing spondylitis within 1 month before this study.

Treatment method: a randomized control method was used. There are 100 patients in the test group, 100 patients in the control group 1, and 100 patients in the control group 2. Test group: the patients were orally administrated with the pharmaceutical product for treating ankylosing spondylitis for combined immunodeficiency according to the present invention prepared in Example 1 twice a day in the morning and evening, and 3 months is a course of treatment. Control group 1: the patients were orally administrated with loxoprofen sodium from Sankyo Co. Ltd., Japan twice a day in the morning and evening, and 3 months is a course of treatment. Control group 2: the patients were orally administrated with the traditional Chinese medicine for treating ankylosing spondylitis prepared in the examples of CN102091137B twice a day in the morning and evening, and 3 months is a course of treatment. No other medications for ankylosing spondylitis is administered during the course of treatment. The indicators for efficacy evaluation in this clinical trial are shown in Table 8.

In particular, the traditional Chinese medicine for treating ankylosing spondylitis prepared in the examples of CN102091137B is prepared as following: weighing the raw materials that contain in the formula ,that is 10 g of rhizoma cibotii, 15 g of pilose antler, 10 g of dipsacus asper, 5 g of scorpio, 15 g of kudzu vine root, 12 g of chuanxiong rhizome, 5 g of caulis spatholobi, 15 g of white paeony root, 15 g of common peony root, 10g of safflower carthamus, 15 g of medicinal cyathula root and 10 g of white mustard seed, adding 1500 ml of water, decocting with slow fire to concentrate the medicine liquid to 250 ml, filtering dregs of a decoction and preparing a decoction.

Table 8 Indicators for efficacy evaluation in this clinical trial

| | | Cure | excellence | upturn | Ineffective |
|---|---|---|---|---|---|
| | Sympt om | Stiffness was disappeared, pain of the sacroiliac and other joints was disappeared, joint swelling was disappeared, and joint movement was free. | Stiffness was obviously improved, pain of the sacroiliac and other joints was obviously improved, joint swelling was obviously improved, and joint movement was obviously improved. | Stiffness was improved, pain of the sacroiliac and other joints was improved, joint swelling was improved, and joint movement was improved. | Stiffness was not improved, pain of the sacroiliac and other joints was not improved, and joint swelling was not changed. |

(continued)

|  | Cure | excellence | upturn | Ineffective |
|---|---|---|---|---|
| Sign | The positive signs of each joint were disappeared and the physiological functions of each joint were returned to normal. | The positive signs of each joint were obviously improved and the physiological functions of each joint were obviously restored. | The positive signs of each joint were Lightened and the physiological functions of each joint have some improvement. | The positive signs of each joint were not improved, and the physiological functions of each joint were not changed. |
| X images | The inflammatory changes of the ligaments and soft tissues around the joints were disappeared, the bone necrosis was absorbed, the new bone in cystic photic zone was formed, the sclerotic bone was absorbed, the trabecular bone structure was reestablished, and the bone mineral density was basically uniform. | The inflammatory changes of the ligaments and soft tissues around the joints were obviously improved, the bone necrosis was mostly absorbed, the new bone in cystic photic zone was mostly formed, the sclerotic bone was mostly absorbed, the trabecular bone structure was partially reestablished, and the bone mineral density was more uniform than that prior to treatment. | The inflammatory changes of the ligaments and soft tissues around the joints were improved, the bone necrosis was partially absorbed, the new bone in cystic photic zone was partially formed, the sclerotic bone was partially absorbed, the trabecular bone structure was slightly reestablished, and the bone mineral density was more uniform than that prior to treatment. | The inflammatory changes of the ligaments and soft tissues around the joints were not improved, the bone necrosis was not absorbed, the new bone in cystic photic zone was not formed, the sclerotic bone was not absorbed. |
| Bone mineral density | The bone mineral density of lumbar vertebra and triangle region was recovered. | The bone mineral density of lumbar vertebra and triangle region was increased. | The bone mineral density of lumbar vertebra and triangle region was increased. | The bone mineral density of lumbar vertebra and triangle region was not changed. |
| Test | Erythrocyte sedimentation rate, C-reactive protein, immunoglobulin, complement, blood routine, antistreptolysin O and other laboratory-related tests were all normal. | One of erythrocyte sedimentation rate, C-reactive protein, immunoglobulin, complement, blood routine, antistreptolysin O and other laboratory-related tests was normal, and two or more of these tests were obviously improved. | One or more of erythrocyte sedimentation rate, C-reactive protein, immunoglobulin, complement, blood routine, antistreptolysin O and other laboratory-related tests were obviously improved. | Erythrocyte sedimentation rate, C-reactive protein, immunoglobulin, complement, blood routine, antistreptolysin O and other laboratory-related tests were not improved. |

After taking one course of treatment and six courses of treatment, the therapeutic effects of the three groups of patients were evaluated. The results are shown in Table 9.

Table 9 Statistical results of clinical trial

|  |  | Cure | excellence | upturn | Ineffective |
|---|---|---|---|---|---|
| Test group | One course of treatment | 0 case | 21 case | 45 case | 34 case |
|  | Three courses of treatment | 15 case | 32 case | 40 case | 13 case |
| Control group 1 | One course of treatment | 0 case | 0 case | 18 case | 82 case |
|  | Three courses of treatment | 0 case | 3 case | 36 case | 61 case |
| Control group 2 | One course of treatment | 0 case | 8 case | 24 case | 68 case |
|  | Three courses of treatment | 5 case | 24 case | 50 case | 21 case |

As seen from Table 9, the administration of the pharmaceutical product for treating ankylosing spondylitis for combined immunodeficiency according to the present invention prepared in Example 1, the administration of loxoprofen sodium from Sankyo Co. Ltd., Japan, and the administration of traditional Chinese medicine for treating ankylosing spondylitis prepared in the examples of CN102091137B can all produce certain positive effects on ankylosing spondylitis, wherein, the patients in the test group administrated with the pharmaceutical product for treating ankylosing spondylitis for combined immunodeficiency according to the present invention prepared in Example 1 has a higher probability of producing a positive effect and the said composition has a better treatment effect and works fastest after taking one course of treatment.

After taking three courses of treatment, all the patients in the three groups stopped taking medication, and whether the symptoms were aggravated or slowed down was observed one month, three months, and twelve months after pharmaceutical product withdrawal. If the symptoms after a period of drug withdrawal were more serious than the symptoms at the time of drug withdrawal, it was labeled as "-", otherwise labeled as "+". Meanwhile, the degree of the difference in symptoms was observed in the manner provided in Table 8. If there were two grades in the difference, it was labeled as "-". For example, if the therapeutic effect of patient A at the time of drug withdrawal was improved, and the therapeutic effect twelve months after drug withdrawal was Ineffective, it was labeled as "-2". The specific results are shown in Table 10.

Table 10 Statistical results of clinical trial after pharmaceutical product withdrawal

|  |  | Cure | Improved | Effective | Ineffective |
|---|---|---|---|---|---|
| Test group | At the time of drug withdrawal | 15 case | 32 case | 40 case | 13 case |
|  | One month after drug withdrawal | 15 case | 32 case | 40 case | 13 case |
|  | three months after drug withdrawal | 15 case | 31 case | 38 case | 16 case |
|  | Twelve months after drug withdrawal | 15 case | 25 case | 36 case | 24 case |
| Control group 1 | At the time of drug withdrawal | 0 case | 3 case | 36 case | 61 case |
|  | One month after drug withdrawal | 0 case | 2 case | 31 case | 67 case |
|  | three months after drug withdrawal | 0 case | 0 case | 20 case | 80 case |
|  | Twelve months after drug withdrawal | 0 case | 0 case | 9 case | 91 case |
| Control group 2 | At the time of drug withdrawal | 5 case | 24 case | 50 case | 21 case |
|  | One month after drug withdrawal | 4 case | 20 case | 45 case | 31 case |
|  | three months after drug withdrawal | 1 case | 14 case | 33 case | 52 case |
|  | Twelve months after drug withdrawal | 0 case | 2 case | 17 case | 81 case |

As seen from Table 9, after taking the pharmaceutical product for treating ankylosing spondylitis for combined immunodeficiency according to the present invention prepared in Example 1 three courses of treatment, loxoprofen sodium from Sankyo Co. Ltd., Japan, and traditional Chinese medicine for treating ankylosing spondylitis prepared in the examples of CN102091137B for three courses of treatment and then withdrawal, the patients in the control group 1 and the control group 2 had more disease relapse, the consolidation of the therapeutic effect could not be ensured. However, after taking the pharmaceutical product for treating ankylosing spondylitis for combined immunodeficiency according to the present invention as prepared in Example 1 and then withdrawal, the therapeutic effect remained basically unchanged, which may be related to the well balanced immune system of patients administrated with the pharmaceutical product for treating ankylosing spondylitis for combined immunodeficiency according to the present invention prepared in Example 1.

The above description is only a preferred embodiment of the present invention, and the scope of protection of the present invention is not limited to the above embodiments. All the technical solutions under the inventive concept fall within the protection scope of the present invention. It should be noted that various modifications and refinements made by those skilled in the art without departing from the principles of the invention are also considered to be within the scope of the invention.

## Claims

1. A pharmaceutical product for treating ankylosing spondylitis for combined immunodeficiency, **characterized in that** the pharmaceutical product for treating ankylosing spondylitis for combined immunodeficiency is prepared from a *Rosa roxbunghii* extract, an *Artemisia argyi* extract, a *hippocampus* extract, and a *Corydalis Rhizoma* extract in a weight ratio of (1-10):(0.5-2):1:(1-10).

2. The pharmaceutical product for treating ankylosing spondylitis for combined immunodeficiency according to claim 1, **characterized in that** the *Artemisia argyi* is *Artemisia absinthium,* and the *Rosa roxbunghii, Artemisia argyi, hippocampus,* and *Corydalis Rhizoma* are all wild.

3. The pharmaceutical product for treating ankylosing spondylitis for combined immunodeficiency according to claim 1, **characterized in that** the weight ratio *of Rosa roxbunghii* extract, *Artemisia argyi* extract, *hippocampus* extract, and *Corydalis Rhizoma* extract is 5:2:1:5.

4. The pharmaceutical product for treating ankylosing spondylitis for combined immunodeficiency according to claim 1 or 2 or 3, **characterized in that** the preparation of the pharmaceutical product is a capsule.

5. A preparation method of the pharmaceutical product for treating ankylosing spondylitis for combined immunodeficiency according to claim 4, **characterized in that** the method comprises the preparation of *Rosa roxbunghii* extract capsule, the preparation of *Artemisia argyi* extract capsule, the preparation of *hippocampus* extract capsule, and the preparation of *Corydalis Rhizoma* extract capsule; the preparation *of Rosa roxbunghii* extract capsule, the preparation of *Artemisia argyi* extract capsule, the preparation of *hippocampus* extract capsule, and the preparation of *Corydalis Rhizoma* extract capsule all include:

   S1, screening and washing raw materials, and softening the clean raw materials by quenching under vacuum;
   S2, subjecting the raw material softened in step S1 to supercritical fluid extraction and separating to obtain an extract liquor and a residue;
   S3, pulverizing and grinding the residue obtained in step S2;
   S4, spray drying the extract liquor obtained in step S2 and adding the pulverized and ground residue obtained in step S3 during the spray drying to obtain a dried raw material extract; and
   S5, filling a formulated amount of the raw material extract obtained in step S4 into a capsule shell to obtain a capsule.

6. A preparation method of the pharmaceutical product for treating ankylosing spondylitis for combined immunodeficiency according to claim 5, **characterized in that**:

   in S1, the method of quenching under vacuum is performed at the temperature of 10-40 °C and the pressure of -0.1 to -0.09 MPa;
   in S2, the fluid for supercritical fluid extraction is carbon dioxide, and the extraction temperature is 30-40 °C;
   in S4, the spray drying temperature is 40-80 °C, the drying time is 3-60 s, and the raw material extract has a particle size of 10-60 $\mu$m; and
   in S5, the capsule shell is a natural plant capsule shell.

7. A preparation method of the pharmaceutical product for treating ankylosing spondylitis for combined immunodeficiency according to claim 3, **characterized in that**:

   the method comprises the preparation of *Rosa roxbunghii* extract capsule, the preparation of *Artemisia argyi* extract capsule, the preparation of *hippocampus* extract capsule, and the preparation of *Corydalis Rhizoma* extract capsule;

the preparation of *Rosa roxbunghii* extract capsule comprises:

S1, screening and washing *Rosa roxbunghii,* and softening the clean *Rosa roxbunghii* by quenching under vacuum; wherein the quenching under vacuum is performed at the temperature of 10-40 °C and the pressure of -0.1 to -0.09 MPa;

S2, subjecting the *Rosa roxbunghii* softened in step S1 to supercritical fluid extraction and separating to obtain a *Rosa roxbunghii* extract liquor and a *Rosa roxbunghii* residue; wherein the fluid for supercritical fluid extraction is carbon dioxide, and the extraction temperature is 30-40 °C;

S3, pulverizing and grinding the *Rosa roxbunghii* residue obtained in step S2;

S4, spray drying the *Rosa roxbunghii* extract liquor obtained in step S2 and adding the pulverized and ground *Rosa roxbunghii* residue obtained in step S3 during the spray drying to obtain a dried *Rosa roxbunghii* extract; wherein the spray drying temperature is 40-80 °C, the drying time is 3-60 s, and the *Rosa roxbunghii* extract has a particle size of 10-60 $\mu$m; and

S5, filling 1-10 parts of *Rosa roxbunghii* extract obtained in the S4 into a natural plant capsule shell to obtain a *Rosa roxbunghii* extract capsule;

the preparation of *Artemisia argyi* extract capsule comprises:

S1, screening and washing *Artemisia argyi,* and softening the clean whole grass of *Artemisia argyi* by quenching under vacuum; wherein the quenching under vacuum is performed at the temperature of 10-40 °C and the pressure of -0.1 to -0.09 MPa;

S2, subjecting the *Artemisia argyi* softened in step S1 to supercritical fluid extraction and separating to obtain an *Artemisia argyi* extract liquor and an *Artemisia argyi* residue; wherein the fluid for supercritical fluid extraction is carbon dioxide, and the extraction temperature is 30-40 °C;

S3, pulverizing and grinding the *Artemisia argyi* residue obtained in step S2;

S4, spray drying the *Artemisia argyi* extract liquor obtained in step S2 and adding the pulverized and ground *Artemisia argyi* residue obtained in step S3 during the spray drying to obtain a dried *Artemisia argyi* extract; wherein the spray drying temperature is 40-80 °C, the drying time is 3-60 s, and the *Artemisia argyi* extract has a particle size of 10-60 $\mu$m; and

S5, filling 0.5-2 parts of *Artemisia argyi* extract obtained in step S4 into a natural plant capsule shell to obtain an *Artemisia argyi* extract capsule;

the preparation of *Hippocampus* extract capsule comprises:

S1, screening and washing *Hippocampus,* and softening the clean *Hippocampus* with viscera and gray-black outer membrane being removed by quenching under vacuum; wherein the quenching under vacuum is performed at the temperature of 10-40 °C and the pressure of -0.1 to -0.09 MPa;

S2, subjecting the *Hippocampus* softened in step S1 to supercritical fluid extraction and separating to obtain a *Hippocampus* extract liquor and a *Hippocampus* residue; wherein the fluid for supercritical fluid extraction is carbon dioxide, and the extraction temperature is 30-40 °C;

S3, pulverizing and grinding the *Hippocampus* residue obtained in step S2;

S4, spray drying the *Hippocampus* extract liquor obtained in step S2 and adding the pulverized and ground *Hippocampus* residue obtained in step S3 during the spray drying to obtain a dried *Hippocampus* extract; wherein the spray drying temperature is 40-80 °C, the drying time is 3-60 s, and the *Hippocampus* extract has a particle size of 10-60 $\mu$m; and

S5, filling 1 part of *Hippocampus* extract obtained in step S4 into a natural plant capsule shell to obtain a *Hippocampus* extract capsule;

the preparation of *Corydalis Rhizoma* extract capsule comprises:

S1, screening and washing *Corydalis Rhizoma,* and softening the clean tuber of *Corydalis Rhizoma* by quenching under vacuum; wherein the quenching under vacuum is performed at the temperature of 10-40 °C and the pressure of -0.1 to -0.09 MPa;

S2, subjecting the *Corydalis Rhizoma* softened in step S1 to supercritical fluid extraction and separating to obtain a *Corydalis Rhizoma* extract liquor and a *Corydalis Rhizoma* residue; wherein the fluid for supercritical fluid extraction is carbon dioxide, and the extraction temperature is 30-40 °C;

S3, pulverizing and grinding the *Corydalis Rhizoma* residue obtained in step S2;

S4, spray drying the *Corydalis Rhizoma* extract liquor obtained in step S2 and adding the pulverized and ground *Corydalis Rhizoma* residue obtained in step S3 during the spray drying to obtain a dried *Corydalis Rhizoma* extract; wherein the spray drying temperature is 40-80 °C, the drying time is 3-60 s, and the *Corydalis Rhizoma* extract has a particle size of 10-60 μm; and

S5, filling 1-10 parts of *Corydalis Rhizoma* extract obtained in step S4 into a natural plant capsule shell to obtain a *Corydalis Rhizoma* extract capsule; and

the *Artemisia argyi* is *Artemisia absinthium,* and the *Rosa roxbunghii, Artemisia argyi, hippocampus,* and *Corydalis Rhizoma* are all wild.

8. Use of the pharmaceutical product for treating ankylosing spondylitis for combined immunodeficiency according to claim 1 in the manufacture of a pharmaceutical product for preventing or treating ankylosing spondylitis.

9. Use of the pharmaceutical product for treating ankylosing spondylitis for combined immunodeficiency prepared by the preparation method according to any of claims 5-7 in the manufacture of a pharmaceutical product for preventing or treating ankylosing spondylitis.

<div align="center">

**INTERNATIONAL SEARCH REPORT**

</div>

| International application No. |
| --- |
| **PCT/CN2016/078650** |

**A. CLASSIFICATION OF SUBJECT MATTER**

A61K 36/738 (2006.01) i; A61K 36/66 (2006.01) i; A61K 36/282 (2006.01) i; A61K 35/60 (2006.01) i; A61K 9/48 (2006.01) i; A61P 19/08 (2006.01) i; A61P 37/04 (2006.01) i

According to International Patent Classification (IPC) or to both national classification and IPC

**B. FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

CNKI, CPRS, WPI, SIPOABS, E Medicine Library: Rosa roxburghii, Fructus Rosae Nomailis, Artemisia, Folium Artemisiae Argyi, woormwood, Artemisia absinthium, Hippocampus, seahorse, Corydalis rhizome, Rhizoma Corydalis, extract, ankylosing spondylitis, immunity, immune, supercritical fluid, spray drying, capsule, drug combination, rosa davurica, Rosa roxburghii Tratt, artemisiae argyi, moxa, Artemisia absinthium, corydalis tuber, corydalis yanhusuo

**C. DOCUMENTS CONSIDERED TO BE RELEVANT**

| Category* | Citation of document, with indication, where appropriate, of the relevant passages | Relevant to claim No. |
| --- | --- | --- |
| A | CN 101396435 A (LIU, Zhimou), 01 April 2009 (01.04.2009), the whole document | 1-9 |
| A | CN 1198945 A (HU, Xiaobo), 18 November 1998 (18.11.1998), the whole document | 1-9 |
| A | YANG, Xuegang; "A Review of Research on Treating Ankylosing Spondylitis in Traditional Chinese Medicine", STUDY JOURNAL OF TRADITIONAL CHINESE MEDICINE, vol. 22, no. 2, 29 February 2004 (29.02.2004), pages 298-299 | 1-9 |

☐ Further documents are listed in the continuation of Box C.  ☒ See patent family annex.

| | |
| --- | --- |
| * Special categories of cited documents:<br><br>"A" document defining the general state of the art which is not considered to be of particular relevance<br><br>"E" earlier application or patent but published on or after the international filing date<br><br>"L" document which may throw doubts on priority claim(s) or which is cited to establish the publication date of another citation or other special reason (as specified)<br><br>"O" document referring to an oral disclosure, use, exhibition or other means<br><br>"P" document published prior to the international filing date but later than the priority date claimed | "T" later document published after the international filing date or priority date and not in conflict with the application but cited to understand the principle or theory underlying the invention<br><br>"X" document of particular relevance; the claimed invention cannot be considered novel or cannot be considered to involve an inventive step when the document is taken alone<br><br>"Y" document of particular relevance; the claimed invention cannot be considered to involve an inventive step when the document is combined with one or more other such documents, such combination being obvious to a person skilled in the art<br><br>"&" document member of the same patent family |
| Date of the actual completion of the international search<br><br>28 December 2016 (28.12.2016) | Date of mailing of the international search report<br><br>**10 January 2017 (10.01.2017)** |
| Name and mailing address of the ISA/CN:<br>State Intellectual Property Office of the P. R. China<br>No. 6, Xitucheng Road, Jimenqiao<br>Haidian District, Beijing 100088, China<br>Facsimile No.: (86-10) 62019451 | Authorized officer<br><br>**LI, Ying**<br><br>Telephone No.: (86-10) **62089874** |

Form PCT/ISA/210 (second sheet) (July 2009)

## INTERNATIONAL SEARCH REPORT
### Information on patent family members

| | International application No. |
|---|---|
| | PCT/CN2016/078650 |

| Patent Documents referred in the Report | Publication Date | Patent Family | Publication Date |
|---|---|---|---|
| CN 101396435 A | 01 April 2009 | None | |
| CN 1198945 A | 18 November 1998 | None | |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102091137 B
- GB 478994 A
- GB 47891594 A